(19) Europäisches Patentamt
European Patent Office
Office européen des brevets

(11) **EP 4 242 656 A1**

(12) **EUROPEAN PATENT APPLICATION**

(43) Date of publication:
**13.09.2023 Bulletin 2023/37**

(21) Application number: **22161450.6**

(22) Date of filing: **10.03.2022**

(51) International Patent Classification (IPC):
*G01N 33/537* *(2006.01)*

(52) Cooperative Patent Classification (CPC):
**G01N 33/5375**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Actome GmbH**
**79110 Freiburg (DE)**

(72) Inventor: **Csaba, Jeney**
**79379 Müllheim (DE)**

(74) Representative: **Mertzlufft-Paufler, Cornelius et al**
**Maucher Jenkins**
**Patent- und Rechtsanwälte**
**Urachstraße 23**
**79102 Freiburg im Breisgau (DE)**

(54) **QUANTITATIVE DETECTION METHOD AND CORRESPONDING DEVICE**

(57) The invention generally suggests a quantitative detection method (1) for a target (2), whereby the target (2), a first probe (3), and a second probe (4) are mixed, wherein the first and second probes (3, 4) bind to the target (2) and wherein a presence of the first probe (3) can be distinguished from a presence of the second probe (4), wherein the mixture (5) of the target (2) and the first and second probes (3, 4) is partitioned in countable compartments (6), wherein a quantity relating to a number of compartments (6), a quantity relating to a number of compartments (6) where at least the first probe (3) is present, a quantity relating to a number of compartments (6) where at least the second probe (4) is present and a quantity relating to a number of compartments (6) where both the first probe (3) and the second probe (4) are present are determined and wherein a quantity relating to a number of compartments (6) that contain the target (2) is determined automatically from the determined quantities (Fig. Fig. 6).

Fig. 6

EP 4 242 656 A1

**Description**

The invention is related to a quantitative detection method for a target, whereby the target, a first probe, and a second probe are mixed, wherein the first and second probes bind to the target and wherein a presence of the first probe can be distinguished from a presence of the second probe.

The invention is further related to a device for quantitative detection of a target.

Quantitative detection methods and/or devices as described before are well known in the state of the art and are particularly important for biological, pharmaceutical and/or clinical applications. Many such methods and/or devices are not able to accurately detect for instance biological specimens of at least low abundance and/or certain molecular interactions that are weak and/or transient. In general, most quantitative detection methods and/or devices do not detect a plethora of targets with a good sensitivity (true-positive rate) and/or specificity (true-negative rate).

A majority of quantitative detection methods require numerous hands-on steps and are thus time consuming and prone to errors which makes such detection methods less suitable for industrial applications. These problems are even more prominent when a sample number is considerably high.

Furthermore, evaluations, especially quantifications of results are often done manually or semi-automatically. Thus, a speed and/or a reproducibility of sample detections are hindered by conventional detection methods and/or devices.

It is therefore the object of the invention to improve quantitative detections.

According to the invention, the features of claim 1 are provided for solving the said object. In particular, in order to solve the aforementioned object in the case of a quantification method of the type described at the beginning, it is thus proposed in accordance with the invention that the mixture of the target and the first and second probes is partitioned in countable compartments. By this, said mixture can be advantageously partitioned allowing multiple parallel measurements at a time. Thus, a detection speed of multiple samples can be accelerated, which may be advantageous.

The determination of the quantities referred to in claim 1 is done preferably by measurement, for example counting.

The mixture may be a liquid mixture, preferably so if the method according to the invention shall quantitively detect a biological, pharmaceutical and/or clinical target of preferably unknown concentration. The mixture might be undiluted and/or diluted. It is to be understood that the mixture can also comprise any conditioning reagents needed to render a target soluble and accessible to detection and quantification.

Of further advantage may be, that at least a single specimen and/or reaction might be detected in a single compartment, thus allowing a good sensitivity of the method.

Compartments might be made of droplets formed by a phase boundary, preferably in which a first fluid is surrounded by a second fluid, as it might be the case in a water-oil emulsion system. Alternatively or additionally compartments may be formed at least partially by a solid element. In general, every compartment type that is known by the expert in the field and/or suitable to detect a target by the method according to the invention might be applicable. Thus, a certain compartment type might be used advantageously for specific detection conditions and/or working conditions.

Counting of the compartments can be done optically. For instance, compartments can be counted optically by counting lattice arrangements in which the compartments are positioned. By this example, a number of the compartments can be determined precisely, which is crucial for the method as further quantifications relate to the number of compartments. The number of compartments can be more than a thousand, preferably more than 20.000 thousand. The number of compartments has, however, no upper limits, if, for instance, generated signals by the method are in a linear range. Such a high number of compartments enables parallel measurements and provides enough data that can be implemented for robust statistical analyses that can improve a quantification result.

It is furthermore proposed in accordance with the invention, that a quantity relating to a number of compartments where at least the first probe is present, a quantity relating to a number of compartments where at least the second probe is present and a quantity relating to a number of compartments where both the first probe and the second probe are present are determined. Since a presence of the first probe can be distinguished from a presence of the second probe, the method allows advantageously to count and calculate simultaneously a number of both probes, that are present in either no compartment, one compartment or in separate compartments. Based on Poisson distribution there might be many double positive compartments containing one or more first and/or second probes. By this example, a quantification of compartments containing a first probe and/or a second probe can be calculated relatively fast and accurate and allows the quantification of the target in said mixture.

It is furthermore proposed in accordance with the invention, that a quantity relating to a number of compartments that contain the target is determined automatically from the determined quantities. An automatic determination of the target based on the aforementioned quantities may be performed relatively fast and accurate, which improves quantitative detections of targets in mixtures.

In an advantageous embodiment of the method described herein, the first probe and/ or the second probe are distinguishable by optical detection. Optical detection of at least one of the aforementioned probes allows a straightforward detection of the probe and thus quantification of compartments containing the probe might be relatively fast and accurate.

The optically detectable probe might contain a marker that can be detected either directly or indirectly. For instance, a preferred marker that is directly detectable might be a fluorophore- a substance that can re-emit light upon light excitation- that is physically connected to the optically detectable probe. An indirectly detectable marker might be a marker that is physically connected to the optically detectable probe. The indirectly detectable marker might change properties of an additional substance that is within the compartment. The additional substance might be optically detectable preferably following a change of its properties caused by the marker. In biological, pharmaceutical and/or clinical applications such an indirect marker might be an enzyme. Another preferable detectable marker might be an amplifiable nucleic acid such as DNA and/or RNA.

Most preferably both the first probe and the second probe are optically detectable and distinguishable.

In an advantageous embodiment of the method described herein, the quantity relating to the number of compartments that contain the target is determined by eliminating false double positive counts originating from compartments that contain the first and second probes without the presence of the target. By this, the quantity of the target in the mixture can be determined more precisely and the sensitivity of the method might be increased.

In an advantageous embodiment of the method described herein, the quantity relating to the number of compartments that contain the target is determined by eliminating double counts from compartments that contain the target with bound first and second probes as well as unbound first and second probes. By eliminating additional such double positive counts caused by unbound first and second probes the quantity of the target in the mixture can be determined more precisely. This is especially advantageous when a target concentration is unknown and very low and the concentration of a first and a second probe is comparably high to concentration of the target.

In an advantageous embodiment of the method described herein, the target, the first probe and/or the second probe are diluted such that they can be described by Poisson distributions. Without ternary complexes (that is a target bound to a first and a second probe), the partitioning of the first and second probes follows a Poisson distribution, and results in calculable number of double coloured counts. Thus, this embodiment is particularly advantageous for correcting errors such as false double positive counts as described before.

A preferable dilution of a mixture might be 1 (mixture): 100,000 (dilution buffer), however, the dilution might depend on the (estimated) concentration of the target. Preferably for a quantification of the target, at least two distinct dilutions of a mixture might be used and analysed.

In an advantageous embodiment of the method described herein, an offset quantity for adjusting the quantity relating to the number of compartments that contain the target is determined. By this, erroneous detection results can be determined and implemented, preferably within the automatic determination of the target quantity, to adjust the quantity relating to the number of compartments that contain the target. Thus, a quantification method can be even more precise and sensitive.

In an advantageous embodiment of the method described herein, the offset quantity is determined by performing the method according to any of the preceding claims without the target. Thus, a target-less offset quantity can be determined. This can be particularly advantageous if at least one of the probes is altered in the presence of the target, even though this probe does not bind to the target and/or if a number of double positive compartments is erroneously but systematically determined. In biological, pharmaceutical and/or clinical applications, such an offset might be determined by a negative-control reaction, preferably parallel to the reaction containing the target.

Alternatively or additionally the offset quantity is determined by performing the method according to any of the preceding claims with a different dilution of the target. By this, the optimal signal-to noise ratio between true double positive counts (signal) and the offset (noise) can be determined, which might be advantageous when low abundant targets need to be quantified.

Both alternatives separately or together might improve the quantification of a target significantly compared to methods known in the art.

In an advantageous embodiment of the method described herein, the target is an antigen. An antigen might be a biopolymer such as polynucleotide, polypeptide, polysaccharide, a combination thereof, or a monomeric subunit that can be used to form said biopolymers.

The antigen might be a biological molecule that is not a biopolymer. Such antigens might be small molecules and/or drugs known preferably to the expert in the fields of molecular biology and pharmacology.

The antigen might at least partially contain a non-natural component.

Alternatively or additionally the first and second probes contain antibodies, preferably marked and/or mutually distinct antibodies. By this, the first and second probes can bind to an antigen and thus the target can be detected and quantified. This embodiment is of particular advantage if the antibodies are marked, especially as described before, in order to optically detect directly or indirectly the probes and/or the target.

Mutually distinct antibodies can preferably contain distinct markers and/or they bind to distinct regions of the antigen and/or the two distinct antibodies do not bind each other. By this, a detection of false double positive counts and/or the detection of false negative counts can be reduced, which can improve the sensitivity and/or the sensibility of the method.

In an advantageous embodiment of the method described herein, the first and second probes bind in a non-overlapping and/or in an independent way to the target. This is particularly advantageous when the first and second probes are mutually distinct antibodies, such as the antibodies described before.

In an advantageous embodiment of the method described herein, a detectability of the first and/ or second probe(s) in the compartment is unaffected by a presence of the target within the compartment. When the target is already bound to the first and/ or second probe(s) additional and unbound first and/or second probes in the compartment might be unaffected by the presence of the target and therefor these unbound probes might not be detected. Thus, a detection of false double positive counts might be reduced. This might particularly increase the sensitivity of the method, especially when, as described before and/or claimed hereafter, the quantity relating to the number of compartments that contain the target is determined by eliminating double counts from compartments that contain the target with bound first and second probes as well as unbound first and second probes.

In an advantageous embodiment of the method described herein, the first and second probes bind to the target through a molecular biological interaction. It is to be understood that any biological interaction known to the expert in the art might lead to an interaction of the target to the first and/or second probe. Therefore, the method can be used preferably in biological, pharmacological and clinical applications, especially if, as described before and/or claimed hereafter, the target is an antigen and/or the first and second probes contain antibodies.

In an advantageous embodiment of the method described herein, the quantity relating to a number of compartments that contain the target is determined using an expression for the probability of a compartment to create a double positive count, in particular wherein the expression comprises a term for a probability of a compartment to contain at least the first and second probes in unbound form. Such an expression for the probability of a compartment to contain a double positive count can preferably be implemented in Poisson distribution since the Poisson model is appropriate for the quantification method, especially when the mixture is diluted as describe before or claimed hereafter and/or the number of compartments is reasonably high. For example, the following equation can be used to determine the probability $(P_0)$ of a compartment to contain a double positive count:

$$P0 = PC\left(1 - e^{-\left(\lambda_{abA}^m - \lambda C\right)}\right)\left(1 - e^{-\left(\lambda_{abB}^m - \lambda C\right)}\right) \qquad Eq.\,1$$

where "$P_C$" designates the probability of complexes calculated by the following equation: $P_c=(1-e^{-\lambda C})$; where "e" designates Euler's number; where "$\lambda C$" is the average number of ternary complexes (that is: targets bound to the first and second probes); where "$\lambda_{abA}^m$" is the average number of unbound first probes (abA) plus a complex consisting of the target bound only to the first probe (abA) and where "$\lambda_{abB}^m$" is the average number of unbound second probe (abB) plus a complex consisting of the target bound only to the second probe (abB). With this equation one can calculate the overlap of complexes (first factor in equation 1) and random double positive counts (second and third factor in equation 1).

Alternatively or additionally the quantity relating to a number of compartments that contain the target is determined using an expression for a probability of a compartment to contain at least the target, preferably reduced by a probability of a compartment to contain both the target and the first and second probes in unbound form $(P_d^m)$. Such an expression can be formulated by the following equation:

$$P_d^m = \left(1 - e^{-\left(\lambda_{abA}^m - \lambda C\right)}\right)\left(1 - e^{-\left(\lambda_{abB}^m - \lambda C\right)}\right) + PC - P0 \qquad Eq.\,2$$

where the terms express the same as for equation 1. Such a solution can either be an exact, preferably analytical, solution, a suitable approximation or which can be solved by a numerical solver.

Either of the equations or both of the equations can be used to determine automatically, as described earlier or as it is claimed hereafter, the absolute count (number) "m" of targets in the mixture with, even if the concentration of the target in the sample is very low. Using an automatic quantification as described herein might improve the sensitivity of the method even further.

The invention is now described in more detail by means of examples of embodiments, but is not limited to these examples of embodiments. Further embodiments result from combining the features of individual or several claims with each other and/or with individual or several features of the embodiments.

Brief description of the drawings:

Fig. 1          depicts a step in the method according to the invention whereby the target , a first probe, and a second probe are mixed, wherein the first and second probes bind to the target,

Fig. 2 and 4    depict steps in the method according to the invention, wherein the mixture of the target, the first and second probes is partitioned in countable compartments and wherein the distinguishable first and second probes are detected. This mixture is the sample,

Fig. 3 and 5    depict steps in the method according to the invention, wherein the mixture of the first and second probes is partitioned in countable compartments and wherein the distinguishable first and second probes are detected. This mixture is the negative-control reaction,

Fig. 6          depicts a step in the method according to the invention wherein a quantity relating to a number of compartments that contain the target is determined automatically from the determined quantities by the method according to the invention,

Fig. 7          depicts a device for quantitative detection of a target, comprising means to carry the method according to the invention.

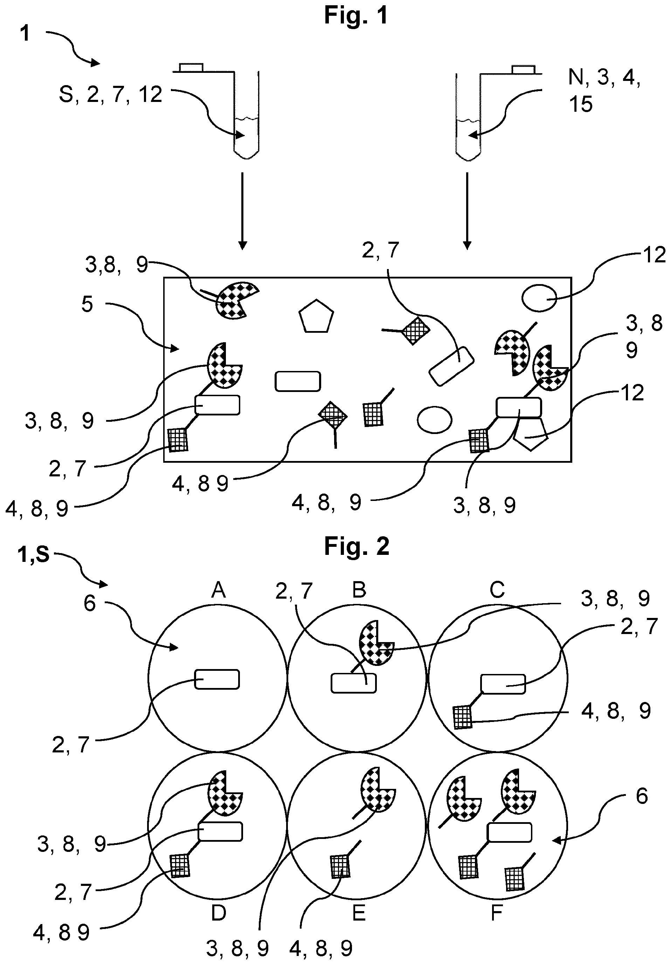

Fig. 1 depicts a step in the method 1 according to the invention whereby the target 2 , a first probe 3 and a second 4 probe are mixed, wherein the first and second probes 3, 4 bind to the target 2.

The biological sample S contains at least target 2, which is an antigen 7 (Fig. 1, upper left panel) to the first probe 3 and the second probes 4. The biological sample S might contain additional molecules that are non-targets 12 (Fig. 1, lower panel), thus the non-targets 12 do not specifically bind to the first probe 3 and the second probes 4. The target 2, 7 might be a biopolymer such as polynucleotide, polypeptide, polysaccharide, a combination thereof, or a monomeric subunit that can be used to form said biopolymers, preferably as describe above.

Both the first and second probes 3, 4 contain an antibody 8. Furthermore both the first probe 3, 8 and the second probes 4, 8 are distinguishable from each other. The first probe 3, 8 and the second probe 4, 8 contain a specific marker 9, which might be a fluorophore and/or an enzyme as described before. The specific marker 9 of the first probe 3, 8 and/or the second probe 4, 8 might also contain a labelled nucleic acid such as DNA and/or RNA that is optically detectable in a direct or indirect manner. In the embodiment described in the figures, the first probe 3, 8 and the second probes 4, 8 are optically detectable and distinguishable through their respective markers 9. In the figures, the marker 9 of the first probe 3, 8 is a "Pacman"-like shape that contains black squares. The marker 9 of the second probe 4, 8 is a chequered rectangle.

Before mixing the sample S with the first probe 3, 8 and the second probe 4, 8 to obtain mixture 5, both probes 3, 4 are present in a separate antibody solution 8, 15 (Fig. 1, upper right panel).

When mixing mixture 5, the first probe 3, 8 and the second probe 4, 8 bind through a molecular biological interaction in a non-overlapping and/or in an independent way to the target 2, 7 (Fig. 1, lower panel). Particularly, a first portion of the antibody solution 8, 15 will be mixed with sample S to obtain mixture 5. A second portion of the antibody solution 8, 15 will be treated as negative-control reaction N, that is the solution to determine the offset quantity by performing the method 1 as described before and/ or as claimed hereafter without the target 2, 7.

A quantitative detection of a target 2, 7 by the method 1 according to the invention might be carried out as described below:
The biological sample S and the first portion of the antibody solution 8, 15 are mixed together and preferably incubated as long as a majority or all of the target 2, 7 are bound to the first 3, 8 and second probe 4,8.

Subsequently to the incubation described in Fig. 1, the target 2, 7, the first probe 3, 8 and the second probe 4, 8 are diluted such that they can be described by Poisson distributions. The mixture 5 of the target 2,7, the first probe 3, 8 and the second probe 4, 8 is partitioned in countable compartments 6 (Fig. 2)

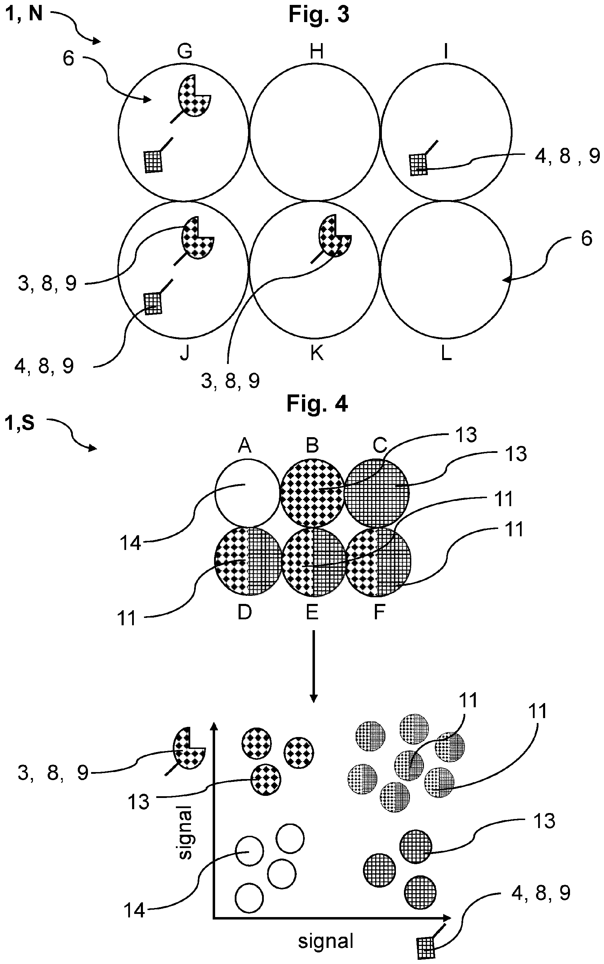

Accordingly, the negative-control reaction N has been diluted so far that the first probe 3, 8 and the second probe 4, 8 can be described by Poisson distributions. The negative-control reaction N containing the first 3, 8 and second probes 4, 8 is partitioned in countable compartments 6 (Fig. 3).

The compartments shown in Fig. 2 and 3 are made of droplets in which a first fluid (water) is surrounded by a second fluid (oil). Alternatively or additionally such compartments 6 may be formed at least partially by a solid element, such as a wall of a microfluidic device. During the quantification of the target 2, 7 by the method 1 according to the invention the number of compartments 6 will be count.

Each of Fig. 2 and 3 depicts exemplary partitions of the target 2, 7 and/or the first probe 3, 8 and/ or the second probe 4, 8 in six different compartments (A-F for sample S, Fig. 2; G-L for negative-control reaction N, Fig. 3). For example, a ternary complex of the target 2, 7, the first probe 3, 8 and the secondary probe 4, 8 can be seen in the compartments D and F in Fig. 2.

It can also be seen, that the first probe 3, 8 and the second probe 4, 8 can colocalise based on Poisson-distribution without the presence of a target 2, 7 (compartment E, Fig. 2 and compartments G and J in Fig. 3) or even with a target 2, 7 (compartment F, Fig. 2). Such colocalizations can lead to false double positive counts and thus to a false quantification of the target 2, 7 quantity in the original sample S.

There can be, based on Poisson-distribution, compartments 6 that either contain no probe or only one kind of a first 3, 8 or second probe 4, 8 (see Fig.2 and Fig. 3).

It is to be understood that Fig. 2 and Fig. 3 only show exemplary partitions and that other partitions will occur in the at least thousand compartments 6 analysed in the method 1 according to the invention.

Depending on the presence of the first 3, 8 and second probe 4, 8 an optical detector (not shown) detects distinguishable signals preferably made by the respective markers of the first 3, 8 and secondary probes 4, 8.

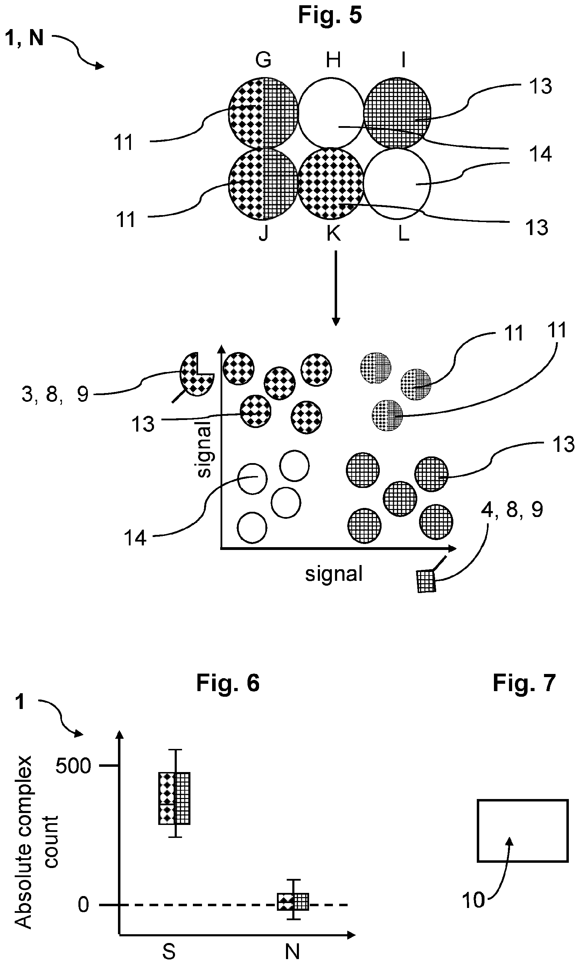

Exemplary signals detectable in the compartments described in Fig. 2 (sample S) and Fig. 3 (negative-control reaction N) are depicted in the respective Fig. 4, upper panel (sample S) and Fig. 5, upper panel (negative-control reaction N).

Double positive counts 11 are detected when both the signal of the first probe 3, 8 and the second probe 4, 8 are detected in one compartment 6. These double positive counts 11 are depicted on the right upper corner of the graphs presented on the bottom of Fig. 4 and Fig. 5, respectively. Accordingly, single counts 13 (either the first 3, 8 or the second 4, 8 probe) and no counts 14 are detected, whereby no counts 14 refers to compartments 6 in which none of the first and second probes 3, 4 has been detected (for instance compartment 6 A in Fig.2).

With these data a quantity relating to a number of compartments 6, a quantity relating to a number of compartments 6 where at least the first probe 3, 8 is present, a quantity relating to a number of compartments 6 where at least the second probe 4, 8 is present and a quantity relating to a number of compartments 6 where both the first probe 3, 8 and the second probe 4, 8 are present are determined and wherein a quantity relating to a number of compartments 6 that contain the target 2, 7 is determined automatically from the determined quantities (Fig. 6).

To quantify the amount of complexes and by this the amount of targets 2, 7 in the sample S (quantity depicted for sample S in Fig. 6), an offset quantity (quantity depicted for negative-control reaction N in Fig. 6) for adjusting the quantity relating to the number of compartments (6) that contain the target (2) is determined. As has been described earlier, the offset quantity (quantity depicted for negative-control reaction N in Fig. 6) was determined by performing the method 1 for the sample S described herein without the target 2, 7 and/ or with a different dilution of the target 2, 7.

The automatically calculated quantity of the target 2, 7 depicted in Fig. 6 is extremely accurate, as the quantity relating to the number of compartments 6 that contain the target 2, 7 is determined by eliminating false double positive counts 11 originating from compartments 6 that contain the first probe 3, 8 and the second probe 4, 8 without the presence of the target 2, 7 (for instance compartment 6 E, Fig. 2) and by eliminating double positive counts 11 from compartments 6 that contain the target 2, 7 with bound first probes 3, 8 and second probes 4, 8 as well as unbound first probes 3, 8 and second probes 4, 8 (for instance compartment 6 F, Fig. 2).

The automatically calculated quantity of the target 2, 7 depicted in Fig. 6 is extremely fast, as the quantity relating to a number of compartments 6 that contain the target 2, 7 is determined using an expression for the probability of a compartment 6 to create a double positive count 11, in particular wherein the expression comprises a term for a probability of a compartment 6 to contain at least the first probe 3, 8 and the second probe 4, 8 in unbound form and/or a term for a probability of a compartment 6 to contain at least the target 2,7 and which is reduced by a probability of a compartment 6 to contain both the target 2, 7 and the first probe 3, 8 and the second probes 4, 8 in unbound form. Such expressions can be equal or similar to equation 1 and equation 2 that were described before.

Fig. 7 depicts a device 10 for quantitative detection of a target, comprising means to carry the method 1 according 1 to the invention. The device 10 has been used to perform the method discussed before and which has been depicted in Fig. 1 to Fig. 6.


EXAMPLES


Example 1. Determination of number of ternary complexes of a target and two DNA labelled antibodies using dPCR. Calculation of number of ternary complexes using empty probabilities.

Antibodies are prepared according to PICOact Antibody Conjugation (aAC) Kit, PICOact Conjugated Antibody Label Loading (aCALL) Kit and two PICO aAC Labels BL and P8 as the commercial protocol. Each antibody carries a 5' nuclease real-time PCR assay amplifiable and detectable single strand DNA label. Generally, the antibodies are free from non-bound labels. Labelled antibodies, at least two antibodies per target having different dPCR detection colours, are incubated with the target forming ternary complexes and diluted to achieve a dilution which results in 0.15 targeted lambda values for both antibodies in the dPCR reaction. dPCR reactions containing the QIAcuity Probe Mix (Qiagen) and universal primers of labels are used to carry out the dPCR amplification. The resulting fluorescence readouts of compartments are thresholded and clustered into empty, single positive and double positives groups. dPCR evaluation is based on general dPCR theory (Basu, A. S. (2017) Digital Assays Part I: Partitioning Statistics and Digital PCR. doi: 10.1177/2472630317705680) providing the single colour based determination of the number of positive compartments (antibody A, B), the number of double coloured compartments and the number of empty compartments.

The following model for the random placement of molecules in compartments is constructed, there are $n_A$ molecules antibody A, and $n_B$ molecules antibody B. In a binding reaction, $n_C$ ternary complex are formed - each ternary complex contains one antibody A, and one antibody B molecule. At equilibrium there are $n_{sA} = n_A - n_C$ unbound antibody A (sA) molecules, $n_{sB} = n_B - n_C$ unbound antibody B (sB) molecules, and $n_C$ ternary complex (C) molecules. The $n_{sA} + n_{sB} + n_C = n_{sA} + n_{sB} - n_C$ molecules are compartmentalised in m compartments, in a way, that each molecule, independently of each other, has equal probability of $1/m$ to fall in each compartment.

The model has the following parameters $\vartheta = (n_{sA}, n_{sB}, n_C)$, or equivalently, $(n_A, n_B, n_C)$. The number of compartments, m, is known.

The vector of the observed data is X = $(X_A, X_B, X_{++})$, where $X_A$ is the number of compartments not containing antibody A, i.e. neither sA, nor C. $X_B$ is the number of compartments not containing antibody B, i.e. neither sB, nor C. $X_{++}$ is the number of compartments not containing both antibodies, i.e. they do not contain C, furthermore at least one of sA and sB is missing. The parameter vector $\vartheta$ is estimated from the observed data X. Method of moments is applied, i.e for the realization of the data

$$\mathbf{X} = \left( x_A, x_B, x_{++} \right)$$

solving the system of equations

$$E_\vartheta(\mathbf{X}) = \mathbf{x}.$$

As the random variable $X_A$ is the sum of (dependent) indicator variables
where $I_{A,i}$ is the indicator of the event that the ith compartment contains neither sA, nor C molecule, the expected value of the sum is the sum of the expected values.

$$E_\vartheta(X_A) = E_\vartheta\left(\sum_{i=1}^{m} I_{A,i}\right) = \sum_{i=1}^{m} E_\vartheta(I_{A,i}) = mE_\vartheta(I_{A,1}).$$

The expected value can be calculated as

$$E_\vartheta(I_{A,1}) = \left(\frac{m-1}{m}\right)^{n_{sA}} \cdot \left(\frac{m-1}{m}\right)^{n_C} = \left(\frac{m-1}{m}\right)^{n_A}.$$

where, if m is large, then this term is approximately equal to $e^{-n_A/m}$.

By introducing the notations, $n_i/m = \lambda_i$ - the average number of type i molecule per compartment (i = sA,sB,C) , $p_i = e^{-\lambda_i}$ - the (approximate) empty probability of i molecule for a compartment (i = sA,sB,C) , $r_i = x_i/m$ - the relative frequency of type i compartment (i = A,B,++), the following equations can be derived

$$p_{sA} p_C = r_A, \quad p_{sB} p_C = r_B.$$

The random variable $X_{++}$ is also the sum of (dependent) indicator variables. The (approximate and empty) probability of the event that a compartment does not contain C, furthermore at least one of sA and sB is missing is (using independence of events) $p_C(p_{sA} + p_{sB} - p_{sA} p_{sB})$ . This gives the equation

$$r_{++} = p_C(p_{sA} + p_{sB} - p_{sA}p_{sB}).$$

Plugging in $p_{sA} = r_A / p_c$ and $p_{sB} = r_B / p_c$, this equation can be solved for $p_c$. The resulting estimates for the (approximate) probabilities pi are given by

$$\hat{p}_{sA} = \frac{r_A + r_B - r_{++}}{r_B},$$

$$\hat{p}_{sB} = \frac{r_A + r_B - r_{++}}{r_A},$$

$$\hat{p}_C = \frac{r_A r_B}{r_A + r_B - r_{++}}.$$

The estimate for the molecule numbers is $\hat{n}_i = -m\ln\hat{p}_i$ (which generally are not integers, and can even be negative). The estimation of $\hat{p}_C$ seems approximately unbiased and the estimates are normally distributed.

Example 2. Alternative method of calculation of number of Ternary complexes using non-empty probabilities.

An equivalent calculation to Example 1. is possible using the data $Y_i = m - X_i$, that is, we measure the number of non-empty compartments containing antibody A, antibody B, and both, respectively (i =A,B,++). Solving the equations $E_\vartheta(Y) = y$ is clearly equivalent to what we have done before. In this case, $E_\vartheta(Y_A) \approx m(1 - p_{sA}p_C)$ and similarly for $Y_B$. Finally,

$$E_\vartheta(Y_{++}) \approx m[(1 - p_{sA})(1 - p_{sB}) + (1 - p_C) -$$
$$- (1 - p_{sA})(1 - p_{sB})(1 - p_C)] =$$
$$= m[1 - p_C(p_{sA} + p_{sB} - p_{sA}p_{sB})].$$

Example 3. Numerical comparison between the Example 1 and Example 2

The following numerical example compares the calculated number of ternary complexes using the two methods of Example 1 and Example 2:

x_{++}= 591 $x_A$= 2652 $x_B$ = 3261 in 25000 compartments (m)
$n_c$, number of ternary complexes calculated according to Example 2 = 124
$n_c$, number of ternary complexes calculated according to Example 1 = 124.

The invention generally suggests a quantitative detection method 1 for a target 2, whereby the target 2, a first probe 3, and a second probe 4 are mixed, wherein the first and second probes 3, 4 bind to the target 2 and wherein a presence of the first probe 3 can be distinguished from a presence of the second probe 4, wherein the mixture 5 of the target 2 and the first and second probes 3, 4 is partitioned in countable compartments 6, wherein a quantity relating to a number of compartments 6, a quantity relating to a number of compartments 6 where at least the first probe 3 is present, a quantity relating to a number of compartments 6 where at least the second probe 4 is present and a quantity relating to a number of compartments 6 where both the first probe 3 and the second probe 4 are present are determined and wherein a quantity relating to a number of compartments 6 that contain the target 2 is determined automatically from the determined quantities. Such a method 1 is of particular advantage for biological, pharmaceutical and/or clinical applications.

List of reference numerals

1        method
2        target
3        first probe

4       second probe
5       mixture
6       compartment
7       antigen
8       antibody
9       marker
10      device
11      double positive count
12      non-target
13      single count
14      no count
15      antibody solution

S       sample
N       negative-control reaction


**Claims**

1.  Quantitative detection method (1) for a target (2), whereby the target (2), a first probe (3), and a second probe (4) are mixed, wherein the first and second probes (3, 4) bind to the target (2) and wherein a presence of the first probe (3) can be distinguished from a presence of the second probe (4), wherein the mixture (5) of the target (2) and the first and second probes (3, 4) is partitioned in countable compartments (6), wherein a quantity relating to a number of compartments (6), a quantity relating to a number of compartments (6) where at least the first probe (3) is present, a quantity relating to a number of compartments (6) where at least the second probe (4) is present and a quantity relating to a number of compartments (6) where both the first probe (3) and the second probe (4) are present are determined and wherein a quantity relating to a number of compartments (6) that contain the target (2) is determined automatically from the determined quantities.

2.  Quantitative detection method (1) according to claim 1, wherein the first probe (3) and/ or the second probe (4) are distinguishable by optical detection.

3.  Quantitative detection method (1) according to any of the preceding claims, wherein the quantity relating to the number of compartments (6) that contain the target (2) is determined by eliminating false double positive counts (11) originating from compartments (6) that contain the first and second probes (3, 4) without the presence of the target (2) .

4.  Quantitative detection method (1) according to any of the preceding claims, wherein the quantity relating to the number of compartments (6) that contain the target (2) is determined by eliminating double positive counts (11) from compartments (6) that contain the target (2) with bound first and second probes (3, 4) as well as unbound first and second probes (3, 4).

5.  Quantitative detection method (1) according to any of the preceding claims, wherein the target (2), the first probe (3) and/or the second probe (4) are diluted such that they can be described by Poisson distributions.

6.  Quantitative detection method (1) according to any of the preceding claims, wherein an offset quantity for adjusting the quantity relating to the number of compartments (6) that contain the target (2) is determined.

7.  Quantitative detection method (1) according to any of the preceding claims, wherein the offset quantity is determined by performing the method (1) according to any of the preceding claims without the target (2) and/ or with a different dilution of the target (2).

8.  Quantitative detection method (1) according to any of the preceding claims, wherein the target (2) is an antigen (7) and/ or wherein the first and second probes (3, 4) contain antibodies (8), preferably marked and/or mutually distinct antibodies.

9.  Quantitative detection method (1) according to any of the preceding claims, wherein the first and second probes (3, 4) bind in a non-overlapping and/or in an independent way to the target (2).

10. Quantitative detection method (1) according to any of the preceding claims, wherein a detectability of the first and/ or second probe(s) (3, 4) in the compartment is unaffected by a presence of the target (2) in the compartment (6).

11. Quantitative detection method (1) according to any of the preceding claims, wherein the first and second probes (3, 4) bind to the target (2) through a molecular biological interaction.

12. Quantitative detection method (1) according to any of the preceding claims, wherein the quantity relating to a number of compartments (6) that contain the target (2) is determined using an expression for the probability of a compartment (6) to create a double positive count , in (11) particular wherein the expression comprises a term for a probability of a compartment (6) to contain at least the first and second probes (3, 4) in unbound form and/or a term for a probability of a compartment (6) to contain at least the target (2), preferably reduced by a probability of a compartment (6) to contain both the target (2) and the first and second probes (3, 4) in unbound form.

13. Quantitative detection method (1) according to any of the preceding claims, wherein the quantity relating to a number of compartments (6) that contain the target (2) is determined by solutions for $\lambda C$ of

$$P_d^m = \left(1 - e^{-\left(\lambda_{abA}^m - \lambda C\right)}\right)\left(1 - e^{-\left(\lambda_{abB}^m - \lambda C\right)}\right) + PC - P0,$$

whereby $P_d^m$ denotes the quantity relating to a number of compartments (6) where both the first probe (3) and the second probe (4) are present, $\lambda_{abA}^m$ and $\lambda_{abB}^m$ denote the quantity relating to a number of compartments (6) where at least the first probe (3) is present and the quantity relating to a number of compartments (6) where at least the second probe (4) is present, respectively, and whereby

$$P0 = PC\left(1 - e^{-\left(\lambda_{abA}^m - \lambda C\right)}\right)\left(1 - e^{-\left(\lambda_{abB}^m - \lambda C\right)}\right)$$

and

$$P_C = \left(1 - e^{-\lambda C}\right).$$

14. Device (10) for quantitative detection of a target (2), comprising means to carry out the method (1) according to any of the preceding claims.

# Fig. 1

1

S, 2, 7, 12

N, 3, 4, 15

3,8, 9

2, 7

12

5

3, 8 9

3, 8, 9

12

2, 7

4, 8 9

4, 8, 9

4, 8, 9

3, 8, 9

# Fig. 2

1,S

6

A     2, 7     B     C

3, 8, 9

2, 7

4, 8, 9

2, 7

3, 8, 9

2, 7

4, 8 9

D

3, 8, 9    4, 8, 9

E

6

F

## Fig. 3

**1, N**

6

G        H        I

4, 8 , 9

6

3, 8, 9

4, 8, 9

J        K        L

3, 8, 9

## Fig. 4

**1,S**

A        B        C

13

13

11

14

11

11

D        E        F

11

11

3, 8, 9

13

signal

14

13

signal

4, 8, 9

## Fig. 5

1, N

G      H      I

13

11

14

11

13

J      K      L

11     11

3, 8, 9

13

signal

13

14

signal

4, 8, 9

## Fig. 6

1

Absolute complex count

500

0

S          N

## Fig. 7

10

# EP 4 242 656 A1

Europäisches
Patentamt
European
Patent Office
Office européen
des brevets

## EUROPEAN SEARCH REPORT

Application Number

EP 22 16 1450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/260277 A1 (ALBERT LUDWIG UNIV FREIBURG [DE]) 30 December 2020 (2020-12-30) * whole document, in particular p. 1, l. 5-7; p. 7, l. 12-18; p. 10, l. 3-5; p. 16, l. 7-11, 29-31; p. 31, l. 3-29; p. 36, l. 9-12, 15-26; p. 38, l. 9-12, 15-22, 24-26, 29-32; p. 38, l. 32 - p. 39, l. 3; claim 11 * | 1-12,14 | INV. G01N33/537 |
| X | EP 3 224 360 A1 (GENEVILLAGE KFT [HU]) 4 October 2017 (2017-10-04) * the whole document * | 1-12,14 | |
| A | US 2011/212848 A1 (DUFFY DAVID C [US] ET AL) 1 September 2011 (2011-09-01) * the whole document * | 1-14 | |
| A | 2472630317705681 ET AL: "Micro-and Nanotechnologies for Quantitative Biology and Medicine", Society for Laboratory Automation and Screening, 1 January 2017 (2017-01-01), pages 387-405, XP055843222, Retrieved from the Internet: URL:https://journals.sagepub.com/doi/pdf/10.1177/2472630317705681 [retrieved on 2021-09-21] * the whole document * | 1-14 | TECHNICAL FIELDS SEARCHED (IPC) G01N |

-/--

The present search report has been drawn up for all claims

| Place of search | Date of completion of the search | Examiner |
|---|---|---|
| The Hague | 25 August 2022 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 22 16 1450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIU HAOMIN ET AL: "A critical review: Recent advances in "digital" biomolecule detection with single copy sensitivity", BIOSENSORS AND BIOELECTRONICS, ELSEVIER SCIENCE LTD, UK, AMSTERDAM , NL, vol. 177, 4 January 2021 (2021-01-04), XP086487015, ISSN: 0956-5663, DOI: 10.1016/J.BIOS.2020.112901 [retrieved on 2021-01-04] * the whole document * | 1 | |

**TECHNICAL FIELDS SEARCHED (IPC)**

The present search report has been drawn up for all claims

| Place of search | Date of completion of the search | Examiner |
|---|---|---|
| The Hague | 25 August 2022 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 22 16 1450

25-08-2022

| Patent document cited in search report | | | Publication date | Patent family member(s) | | Publication date |
|---|---|---|---|---|---|---|
| WO 2020260277 | A1 | | 30-12-2020 | CA | 3143616 A1 | 30-12-2020 |
| | | | | CN | 114041055 A | 11-02-2022 |
| | | | | EP | 3990919 A1 | 04-05-2022 |
| | | | | IL | 289149 A | 01-02-2022 |
| | | | | KR | 20220025829 A | 03-03-2022 |
| | | | | WO | 2020260277 A1 | 30-12-2020 |
| EP 3224360 | A1 | | 04-10-2017 | CA | 2968488 A1 | 02-06-2016 |
| | | | | CN | 107430130 A | 01-12-2017 |
| | | | | EP | 3224360 A1 | 04-10-2017 |
| | | | | ES | 2763563 T3 | 29-05-2020 |
| | | | | JP | 6871168 B2 | 12-05-2021 |
| | | | | JP | 2017536846 A | 14-12-2017 |
| | | | | KR | 20170086627 A | 26-07-2017 |
| | | | | SG | 10202008300R A | 29-10-2020 |
| | | | | SG | 11201703689S A | 29-06-2017 |
| | | | | US | 2017269098 A1 | 21-09-2017 |
| | | | | US | 2021003585 A1 | 07-01-2021 |
| | | | | WO | 2016083793 A1 | 02-06-2016 |
| US 2011212848 | A1 | | 01-09-2011 | CA | 2791654 A1 | 09-09-2011 |
| | | | | CN | 102884431 A | 16-01-2013 |
| | | | | CN | 104820092 A | 05-08-2015 |
| | | | | EP | 2542891 A2 | 09-01-2013 |
| | | | | EP | 2995955 A1 | 16-03-2016 |
| | | | | EP | 3330709 A1 | 06-06-2018 |
| | | | | EP | 3943941 A1 | 26-01-2022 |
| | | | | ES | 2544440 T3 | 31-08-2015 |
| | | | | ES | 2659786 T3 | 19-03-2018 |
| | | | | ES | 2886945 T3 | 21-12-2021 |
| | | | | JP | 5551798 B2 | 16-07-2014 |
| | | | | JP | 5860922 B2 | 16-02-2016 |
| | | | | JP | 2013521499 A | 10-06-2013 |
| | | | | JP | 2014170011 A | 18-09-2014 |
| | | | | US | 2011212848 A1 | 01-09-2011 |
| | | | | US | 2012277114 A1 | 01-11-2012 |
| | | | | US | 2012289428 A1 | 15-11-2012 |
| | | | | US | 2018017552 A1 | 18-01-2018 |
| | | | | US | 2019302109 A1 | 03-10-2019 |
| | | | | US | 2020393457 A1 | 17-12-2020 |
| | | | | WO | 2011109364 A2 | 09-09-2011 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BASU, A. S.** *Digital Assays Part I: Partitioning Statistics and Digital PCR,* 2017